# EUROPEAN PATENT SPECIFICATION

(11) **EP 1 403 363 B1**
(45) Date of publication and mention of the grant of the patent: **24.02.2010**
(21) Application number: 03103435.8
(22) Date of filing: 17.09.2003
(51) Int. Cl.: C12M 3/00, C12M 1/04, C12M 1/34, C12M 1/36

(54) **CO2 incubator**
CO2 Inkubator
Incubateur à CO2

(30) Priority: 27.09.2002 JP 2002282676
(43) Date of publication of application: 31.03.2004
(73) Proprietor: SANYO Electric Co., Ltd., Moriguchi-shi Osaka 570-8677 (JP)
(72) Inventor: Tamaoki, Yuichi, Gunma (JP); Busujima, Hiroki, Gunma (JP); Osawa, Shinji, Gunma (JP); Kikuchi, Yasuhiro, Gunma (JP)
(74) Representative: Piotrowicz, Pawel Jan Andrzej

(56) References cited:
- WO-A-01/84096
- GB-A- 2 138 949
- US-A- 3 929 584
- US-A- 4 892 830
- US-A- 6 010 243
- US-A- 6 117 687

## Description

The present invention relates to a CO₂ incubator for incubating cells sampled from blood or a specimen by controlling a temperature, a humidity and an atmosphere of the cells.

In recent years, with the development of fields regarding biotechnology and regeneration medicine, works for incubating cells by using an incubator tend to increase. To accelerate the incubation of the cells, it is necessary to regulate an incubation space suitable for each cell, and heretofore, some incubators have been developed which control a temperature, a humidity and an atmosphere in the incubation space.

WO-A-0184096 describes a gas measurement system for measuring the concentration of CO₂ gas in an incubator. The gas measurement system includes a reference detector and a sample detector.

US6117687 uses a microprocessor to control the CO₂ level inside the incubator.

GB-A-2 138 949 describes a technique for calibrating a CO₂ sensor. The sensor includes a reference portion and a measuring portion.

Particularly, to incubate the cells which requires severe concentration conditions of a CO₂ (carbon dioxide) gas, a CO₂ incubator is used as a device for controlling the CO₂ gas concentration in the incubation space in addition to a device for controlling the temperature and the humidity (e.g., refer to Patent Document 1 and Patent Document 2).
[Patent Document 1]
   Official gazette of Japanese Patent Application Laid-Open No. 9-23877
[Patent Document 2]
   Official gazette of Japanese Patent Application Laid-Open No. 2000-93156

However, in the case of the conventional CO₂ incubator, when a door is automatically or manually opened and closed, CO₂ leaks to the outside from a storeroom of the CO₂ incubator and the CO₂ gas concentration in the storeroom fluctuates. Moreover, when the door is frequently opened and closed to put in or take out the incubation cells, the CO₂ gas concentration in the storeroom fluctuates before the CO₂ gas concentration in the storeroom returns to its predetermined concentration. Therefore, there is a problem that the state of the incubation space of the cells becomes unstable, which adversely affects the growth of the cells.

For the solution of the problem, in the case of the conventional CO₂ incubator, a CO₂ concentration sensor is disposed in the storeroom for the fluctuation of the CO₂ gas concentration. In consequence, when the CO₂ concentration is recognized to be lower than a set value in accordance with an output of the sensor, a switching valve for supplying the CO₂ gas into the storeroom is opened, and when the CO₂ concentration reaches the set value, the switching valve is closed.

In this case, the above conventional CO₂ gas concentration sensor has a poor concentration detecting performance, and hence, a problem that the accuracy of the detected concentration is low, and moreover, a long time is required to detect the concentration. In addition, an airtight structure is used to reduce a gas consumption, and if overshoot occurs, it takes a long time to return to a predetermined value. Therefore, there is no way other than an operation of decreasing a gas injection quantity to decelerate recovery. The above control is due to an imperfect performance of the sensor. In any case, when the switching valve for supplying the CO₂ gas is controlled in accordance with the output of the CO₂ gas concentration sensor as in the conventional case, a problem occurs that the actual CO₂ gas concentration overshoots or undershoots to the preset CO₂ gas concentration.

Therefore, there is a problem that it is difficult to realize the strict CO₂ gas concentration and it is impossible to sufficiently regulate the cell incubation space.

According to a first aspect of the present invention, there is provided a CO₂ incubator for incubating a culture medium accommodated in an incubation space defined in a storeroom, the CO₂ incubator comprising: CO₂ gas concentration detection means for detecting a CO₂ concentration in the incubation space; CO₂ gas supply means for supplying CO₂ gas into the incubation space; and control means for controlling the CO₂ gas supply means, further comprising CO₂ gas concentration setting means for setting a CO₂ gas concentration; the control means being configured to execute an operation of proportion, proportion and integration, or proportion and integration and differentiation on the basis of a deviation between the detected CO₂ gas concentration and the set CO₂ gas concentration value to calculate a CO₂ gas supply time per unit time to the incubation space and a stop time, wherein said proportion operation calculates a control amount in proportion to the deviation for reducing the deviation, said integral operation calculates a control amount for reducing an integrated value of the deviation and said differential operation calculates a control amount for reducing a differentiated value of the deviation; and the control means being configured to control supply of CO₂ gas to the incubation space from the CO₂ gas supply means in accordance with the calculated supply time and stop time.

Thus, a CO₂ incubator can accurately control a CO₂ gas concentration in an incubation space, and quickly cope with a sudden change of the CO₂ gas concentration, and avoid overshoot and undershoot of CO₂ gas.

In consequence, even if the CO₂ gas concentration in the incubation space is extremely changed by opening or closing a door, the CO₂ gas can be quickly supplied to the incubation space in accordance with the changed CO₂ gas concentration in the incubation space, whereby the stable incubation space can be provided.

The CO₂ gas concentration detection means may be constituted of a CO₂ sensor using infrared rays.

A plurality of incubation spaces may be disposed and the control means may select the gas in any incubation space, detect the CO₂ gas concentration of the selected gas by the CO₂ gas concentration detection means, and control the supply of the CO₂ gas to each incubation space in accordance with the detected CO₂ gas concentration.

This can allow the CO₂ gas concentration can be controlled for each incubation space.

Moreover, since the CO₂ gas concentration detection means and the control means can control the CO₂ gas concentrations in the respective incubation spaces by using common means, it is possible to avoid a fluctuation of the CO₂ gas concentration in each incubation space caused by an error of the CO₂ gas concentration detection means or the control means, as compared with a case where the CO₂ gas concentrations in the respective incubation spaces are controlled by the plurality of CO₂ incubators.

The control means may display the CO₂ gas concentration detected in each incubation space.

This can allow the CO₂ gas concentration in each incubation space to be easily visually confirmed, whereby convenience is further improved.

According to a second aspect of the present invention, there is provided a CO₂ gas controller for controlling supply of CO₂ gas to a given space, the controller comprising: means for receiving a value of CO₂ gas concentration in said given space; and means for outputting a signal to means for supplying CO₂ gas to said given space; said controller being configured to execute an operation of proportion, proportion and integration, or proportion, integration and differentiation on the basis of a difference between said value of CO₂ gas concentration and a set value of CO₂ gas concentration so thereby to determine a CO₂ gas supply time and stop time and to output control signals in accordance with said supply time and stop time, wherein said proportion operation is for calculating a control amount in proportion to the deviation for reducing the deviation, said integral operation is for calculating a control amount for reducing an integrated value of the deviation and said differential operation is for calculating a control amount for reducing a differentiated value of the deviation.

According to a third aspect of the present invention, there is provided a method of controlling supply of CO₂ gas to a given space, the method comprising: receiving a value of CO₂ gas concentration in said given space; executing an operation of proportion, proportion and integration, or proportion, integration and differentiation on the basis of a difference between the measured value of CO₂ gas concentration and a set value of CO₂ gas concentration so thereby to determine a CO₂ gas supply time and stop time; outputting control signals to means for supplying CO₂ gas to said given space in accordance with said supply time and stop time, wherein said proportion operation is for calculating a control amount in proportion to the deviation for reducing the deviation, said integral operation is for calculating a control amount for reducing an integrated value of the deviation and said differential operation is for calculating a control amount for reducing a differentiated value of the deviation.

According to a fourth aspect of the present invention, there is provided a computer program comprising instructions which, when executed by data processing means, causes said data processing means to perform the method.

According to a fifth aspect of the present invention, there is provided a computer program product including the computer program.
FIG. 1 is a schematic block diagram of a CO₂ incubator of the present invention showing the flow of air; and
FIG. 2 is a schematic block diagram of a CO₂ incubator of another embodiment showing the flow of air.

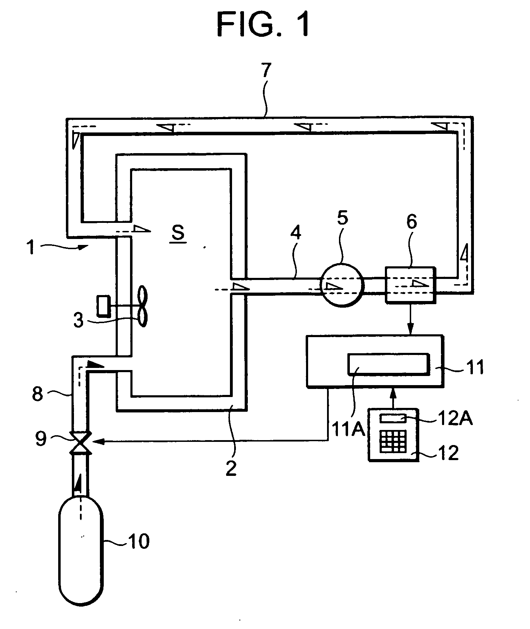

Next, an embodiment of the present invention will be described below in detail by referring to the accompanying drawings. FIG. 1 shows a schematic block diagram off a CO₂ incubator 1 of the present invention showing the flow of air. In the case of the CO₂ incubator 1 of the present invention, a body 2 is constituted of an adiabatic housing having an opening (not shown) on, for example, one face, and an incubation space S is defined in the body 2 (in the storeroom). Moreover, the body 2 is provided with a door, not shown, for closing the opening which can be opened and closed.

The body 2 is provided with an air-agitating blower 3 for agitating the air in the incubation space S to uniform the state of the air. It is to be noted that the air-agitating blower 3 is operated by a blower motor 3A, and the blower motor 3A is controlled by a controller not shown.

Moreover, the body 2 is connected to a measurement air sampling tube 4 so as to communicate with the inside of the incubation space S, and the measurement air sampling tube 4 is connected to a CO₂ gas concentration sensor 6 as CO₂ gas concentration detection means for detecting the CO₂ gas concentration in the incubation space S through a pump 5. The CO₂ gas concentration sensor 6 used in this embodiment may be a CO₂ sensor using infrared rays.

This CO₂ sensor using the infrared rays calculates the CO₂ gas concentration by using a principle that the CO₂ gas absorbs a wavelength of 4.3 µm. That is to say, the CO₂ sensor measures a wavelength absorbing degree, converts the measured data into an electrical signal, and calculates the CO₂ gas concentration. Moreover, this CO₂ sensor (CO₂ gas concentration sensor 6) may be connected to a CO₂ gas controller 11 which will be described later in detail.

Furthermore, the CO₂ gas concentration sensor 6 is connected to a measurement air return tube 7 whose one end communicates with the inside of incubation space S of the body 2. In consequence, when the pump 5 is operated, the air taken by the CO₂ gas concentration sensor 6 through the measurement air sampling tube 4 from the inside of the incubation space S is returned to the inside of the incubation space S through the measurement air return tube 7.

On the other hand, the body 2 is connected to a CO₂ gas supply tube 8 so as to communicate with the inside of the incubation space S, and the CO₂ gas supply tube 8 is connected to a CO₂ gas cylinder 10 through an electromagnetic switching valve 9 as CO₂ gas supply means. In this CO₂ gas cylinder 10, the CO₂ gas having a purity of 95% or more may be contained.

Here, the CO₂ gas controller 11 will be described below. The input side of the CO₂ gas controller 11 is connected to the CO₂ gas concentration sensor 6 and a control panel 12, and the output side of the CO₂ gas controller 11 is connected to the electromagnetic switching valve 9.

The control panel 12 is provided with CO₂ gas concentration setting means for setting the CO₂ gas concentration in the incubation space S, and for example, the control panel 12 is disposed in front of the body 2. Moreover, the control panel 12 may be provided with a display portion 12A for displaying the actually detected CO₂ gas concentration in the incubation space S and the set CO₂ gas concentration.

The CO₂ gas controller 11 controls the electromagnetic switching valve 9 as CO₂ supply means in accordance with the CO₂ gas concentration sensor 6 and control panel 12 and includes a PID-operation processing section 11A. The PID-operation processing section 11A executes operations of proportion (P), integration (I) and differentiation (D) on the basis of a deviation e between a CO₂ gas concentration in the incubation space S detected by the CO₂ gas concentration sensor 6 and a set CO₂ gas concentration value which is optionally set by the control panel 12. That is, the PID-operation processing section 11A performs a proportional operation for calculating a control amount so as to reduce the deviation e in proportion to the deviation e between the CO₂ gas concentration detected by the CO₂ gas concentration sensor 6 and a set CO₂ gas concentration value, an integral operation for calculating a control amount for reducing an integrated value of the deviation e, and a differential operation for calculating a control amount for reducing a gradient (differentiated value) of a change of the deviation. Next, these control amounts are added together to calculate a CO₂ gas supply time per unit time (every certain cycle of, e.g., 3 seconds) of the electromagnetic switching valve 9 and a stop time in accordance with the control amounts.

Then, the CO₂ gas controller 11 controls the electromagnetic switching valve 9 as CO₂ gas supply means in accordance with the CO₂ gas supply time and the stop time calculated in accordance with the PID control and controls the supply of the CO₂ gas to the incubation space S from the CO₂ gas cylinder 10. In the case of this embodiment, operation processings of proportion, integration, and differentiation are performed in accordance with a deviation between a detected CO₂ gas concentration and a set CO₂ gas concentration set value to calculate a CO₂ gas supply time and a stop time. Moreover, it is allowed to calculate the CO₂ gas supply time and the stop time by executing operations of only proportion or operations of only proportion and integration in accordance with the deviation.

Operations of a CO₂ incubator will be described below in accordance with the above configuration. First, a user operates the control panel 12 to set the CO₂ gas concentration in the incubation space S. In this case, some air in the incubation space S is attracted into the measurement air sampling tube 4 by operating the pump 5 and captured into the CO₂ gas concentration sensor 6. Thereafter, the air used for measurement is returned to the incubation space S through the measurement air return tube 7.

In this case, the CO₂ gas concentration sensor 6 measures the absorbance of a wavelength of 4.3 µm by infrared rays to calculate a CO₂ gas concentration. The CO₂ gas controller 11 executes the above-described PID operation processing in accordance with the calculated CO₂ gas concentration and the CO₂ gas concentration set value set as described above. Moreover, the controller 11 calculates the CO₂ gas supply time and the stop time per unit time in accordance with the PID operation processing and controls the electromagnetic switching valve 9 in accordance with the supply time and the stop time. Then, the controller 11 supplies the CO₂ gas into the incubation space S through the CO₂ gas supply tube 8 from the CO₂ gas cylinder 10. The CO₂ gas supply quantity increases when the rate of the supply time in the above three secconds (supply time + stop time) rises but decreases when the rate lowers. The above operation is calculated every three sec to perform a fine control.

Thereby, it is possible to prevent overshoot and undershoot in the control of a CO₂ gas concentration and accurately control the CO₂ gas concentration in the incubation space S. Therefore, even if the CO₂ gas concentration in the incubation space S is extremely changed by opening or closing the door, it is possible to quickly supply the CO₂ gas into the incubation space S in accordance with a changed CO₂ gas concentration in the incubation space S and stably supply the incubation space S.

Particularly, because the CO₂ gas concentration sensor 6 of this embodiment for detecting the CO₂ gas concentration in the incubation space S is constituted of a CO₂ sensor using infrared rays, it is possible to further quickly and accurately detect the CO₂ gas concentration in the incubation space S.

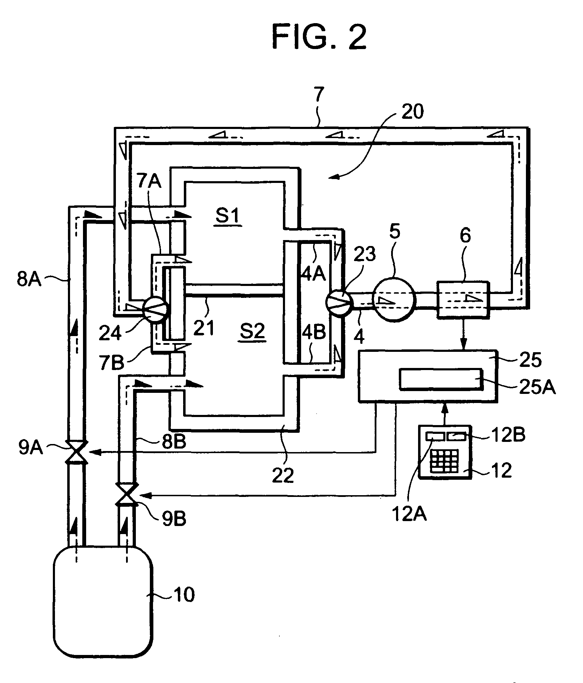

Then, another embodiment of the present invention is described below by referring to FIG. 2. FIG. 2 shows a schematic block diagram of a CO₂ incubator 20 of another embodiment of the present invention showing the flow of air. It is to be noted that the members having the same symbols as in FIG. 1 have similar effects.

In the case of the CO₂ incubator 20 of this embodiment, a body 22 is constituted of an adiabatic housing having an opening (not illustrated) on one face the same as the case of the above embodiment. Moreover, a partition wall 22 is formed in the inside (storeroom) of the body 22 and incubation spaces 1S and 2S divided by the partition wall 21 are also formed. Furthermore, the body 22 is provided with a not-illustrated door for blocking the incubation spaces 1S and 2S respectively so that the opening can be opened or closed.

On the other hand, the body 22 is connected with measurement air sampling tubes 4A and 4B so as to communicate with insides of the incubation spaces S1 and S2, respectively, and these measurement air sampling tubes 4A and 4B are connected to a measurement air sampling tube 4 through a three-way tube 23. The measurement air sampling tube 4 connected to a CO₂ gas concentration sensor 6 as CO₂ gas concentration detection means for detecting the CO₂ gas concentration in the incubation space S1 of S2 through a pump 5. Also in the case of this embodiment, the CO₂ gas concentration sensor 6 may be a CO₂ sensor using infrared rays. Moreover, the CO₂ gas concentration sensor 6 may be connected to a CO₂ gas controller 25 which will be described later in detail.

Furthermore, the CO₂ gas concentration sensor 6 is connected to a measurement air return tube 7, and the other end of the measurement air return tube 7 is connected to measurement air return tubes 7A and 7B communicating with the incubation spaces S1 and S2 through a three-way tube 24. In consequence, when the pump 5 is operated, the air selectively captured into the measurement air sampling tube 4 from the incubation space S1 or S2 is returned to the original incubation space S1 or S2 through the CO₂ gas concentration sensor 6 and measurement air return tube 7.

Furthermore, the body 22 is connected to CO₂ gas supply tubes 8A and 8B so as to communicate with the insides of the incubation spaces S1 and S2, and the CO₂ gas supply tubes 8A and 8B are connected to a CO₂ gas cylinder 10 through electromagnetic switching valves 9A and 9B as CO₂ gas supply means.

The CO₂ gas controller 25 will be described below. The input side of the CO₂ gas controller 25 is connected to the CO₂ gas concentration sensor 6 and control panel 12, and the output side of the CO₂ gas controller 11 is connected to the three-way valves 23 and 24 and the electromagnetic switching valves 9A and 9B.

The control panel 12 serves as CO₂ gas concentration setting means for setting the CO₂ gas concentration in each of the incubation spaces S1 and S2 the same as the case of the above embodiment and is set to, for example, the front of the body 2. Furthermore, the control panel 12 may be provided with display portions 12A and 12B for displaying an actually detected CO₂ gas concentration in each of the incubation spaces S1 and S2 and a set CO₂ gas concentration.

The CO₂ gas controller 25 includes a PID-operation processing section 25A therein as in the CO₂ gas controller 11 of the above embodiment, and controls the electromagnetic switching valve 9A or 9B as the CO₂ supply means by the CO₂ gas concentration sensor 6 for detecting the CO₂ gas concentration of the air in selected one of the incubation spaces S1 and S2 and the control panel 12 as the CO₂ gas concentration setting means. It is to be noted that the PID-operation processing section 25A may have the same constitution as the PID-operation processing section 11A of the above embodiment.

Operations of the CO₂ incubator 20 of the present invention will be described below in accordance with the above configuration. First, a user operates the control panel 12 to set the CO₂ gas concentration in the incubation space S1 and/or S2. The CO₂ gas concentration controller 25 selects either of the incubation spaces S1 and S2 and opens one of the three-way valves 23 and 24 and closes the other so as to make it possible to sample the air in the selected incubation space S1 or S2.

Thereafter, some of the air in the selected incubation space S1 or S2 is attracted into the measurement air sampling tube 4 by operating the pump 5 and captured into the CO₂ gas concentration sensor 6. Then, the air used for measurement is returned to the original incubation space S1 or S2 through the measurement air return tube 7.

In this case, the CO₂ gas concentration sensor 6 measures the absorbance of a wavelength of 4.3 µm with infrared rays and calculates a CO₂ gas concentration. Then, the CO₂ gas controller 25 performs the PID control the same as the case of the above embodiment in accordance with the calculated CO₂ gas concentration and a preset CO₂ gas concentration set value, calculates the CO₂ gas supply time and the stop time for each based unit time, and controls the electromagnetic switching valve 9A or 9B corresponding to the selected incubation space S1 or S2 in accordance with the calculated supply time and stop time. Moreover, the controller 25 supplies the CO₂ gas to the incubation space S1 or S2 from the CO₂ gas cylinder 10 through the CO₂ gas supply tube 8A or 8B.

According to the above configuration, it is possible to avoid overshoot or undershoot through the control of the CO₂ gas concentration in each of the incubation spaces S1 and S2 and accurately control the CO₂ gas concentration in each of the incubation spaces S1 and S2. Therefore, even if the CO₂ gas concentration in the incubation spaces S1 and S2 is extremely changed by opening or closing the door, it is possible to quickly supply the CO₂ gas to the incubation spaces S1 and S2 in accordance with the changed CO₂ gas concentration in each of the incubation spaces S1 and S2 and provide stable incubation spaces S1 and S2.

Moreover, even in the case of the CO₂ incubator 20 in which a plurality of incubation spaces are defined as in this embodiment, the CO₂ gas concentration in each of the incubation spaces S1 and S2 can be controlled by using the common pump 5, the CO₂ gas concentration sensor 6 and the CO₂ gas controller 25, whereby a plurality of types of incubation spaces can be defined in one CO₂ incubator 20.

Particularly in the above case, because a CO₂ gas concentration can be controlled by using the common CO₂ gas concentration sensor 6 and CO₂ gas controller 25, it is possible to avoid the fluctuation of the CO₂ gas concentration in a incubation space caused by an error of CO₂ gas concentration detection means or control means compared to the case of controlling the CO₂ gas concentration in each of the incubation spaces S1 and S2 by a plurality of CO₂ incubators.

Moreover, because the control panel 12 of this embodiment is provided with the display portions 12A and 12B for displaying the CO₂ gas concentrations detected in the respective incubation spaces S1 and S2, the CO₂ gas concentrations in the respective incubation spaces S1 and S2 can be easily visually confirmed, whereby convenience is further improved.

With regard to the CO₂ incubators 1 and 20 of the above embodiments, reference has been made to the CO₂ gas concentration control alone in the incubation spaces S1 and S2. However, it is also allowed to use an incubator making it possible to control an environment required to incubate cells such as temperature control and humidity control in each of the incubation spaces S1 and S2.

As described above, according to the present invention, a CO₂ incubator for incubating a culture medium accommodated in an incubation space defined in a storeroom comprises CO₂ gas concentration detection means for detecting a CO₂ concentration in the incubation space, CO₂ gas concentration setting means for setting the CO₂ concentration in the incubation space, CO₂ gas supply means for supplying the CO₂ gas into the incubation space, and control means for controlling the CO₂ gas supply means, wherein the control means executes an operation of proportion, proportion and integration, or proportion and integration and differentiation on the basis of a deviation between the CO₂ gas concentration in the incubation space and a set CO₂ gas concentration value by the CO₂ gas concentration detection means and the CO₂ gas concentration setting means to calculate a CO₂ gas supply time per unit time to the incubation space and a stop time, and supplies the CO₂ gas to the incubation space from the CO₂ gas supply means in accordance with the calculated supply time and stop time. Accordingly, overshoot and undershoot of the CO₂ gas concentration can be previously avoided, whereby the CO₂ gas concentration can be accurately controlled.

Consequently, even if the CO₂ gas concentration in the incubation space is extremely changed, e.g., by opening or closing a door, the CO₂ gas can be quickly supplied to the incubation space in accordance with the changed CO₂ gas concentration in the incubation space, whereby the stable incubation space can be provided.

According to the invention of claim 2, the CO₂ gas concentration detection means is constituted of a CO₂ sensor using infrared rays in the invention of claim 1, and hence, the CO₂ gas concentration in the incubation space can be further quickly and accurately detected.

According to the invention of claim 3, a plurality of incubation spaces are disposed, and the control means selects any gas in any incubation space, detects the CO₂ gas concentration of the selected gas by the CO₂ gas concentration detection means, and controls the supply of the CO₂ gas to each incubation space in accordance with the detected CO₂ gas concentration. Accordingly, it is possible to control the CO₂ gas concentration in each incubation space.

Moreover, because the CO₂ gas concentration detection means and the control means control the CO₂ gas concentration in each incubation space by using common means, it is possible to previously avoid the fluctuation of the CO₂ gas concentration in an incubation space caused by an error of the CO₂ gas concentration detection means or the control means, as compared with the case of controlling CO₂ gas concentrations in the incubation spaces by a plurality of CO₂ incubators.

According to the invention of claim 4, the control means displays the CO₂ gas concentration detected in each incubation space in the invention of claim 3, and hence, the CO₂ gas concentration in each incubation space can be easily visually confirmed, whereby convenience is further improved.

## Claims

1. A CO₂ incubator (1) for incubating a culture medium accommodated in an incubation space (S) defined in a storeroom, the CO₂ incubator (1) comprising:
CO₂ gas concentration detection (6) means for detecting a CO₂ concentration in the incubation space (S);
CO₂ gas supply means (8, 9, 10) for supplying CO₂ gas into the incubation space (S); and
control means (11) for controlling the CO₂ gas supply means (9),
**characterised by**:
CO₂ gas concentration setting means (12) for setting a CO₂ gas concentration;
the control means (11) being configured to execute an operation of proportion, proportion and integration, or proportion and integration and differentiation on the basis of a deviation between the detected CO₂ gas concentration and the set CO₂ gas concentration value to calculate a CO₂ gas supply time per unit time to the incubation space and a stop time, wherein said proportion operation calculates a control amount in proportion to the deviation for reducing the deviation, said integral operation calculates a control amount for reducing an integrated value of the deviation and said differential operation calculates a control amount for reducing a differentiated value of the deviation; and
the control means (11) being configured to control supply of CO₂ gas to the incubation space (S) from the CO₂ gas supply means (8, 9, 10) in accordance with the calculated supply time and stop time.

2. The CO₂ incubator (1) according to claim 1, wherein the CO₂ gas concentration detection means (6) is constituted of a CO₂ sensor using infrared rays.

3. The CO₂ incubator according to claim 1 or 2, wherein a plurality of incubation spaces (S1, S2) are disposed and the control means (25) selects the gas in any incubation space, detects the CO₂ gas concentration of the selected gas by the CO₂ gas concentration detection means (6), and controls the supply of the CO₂ gas to each incubation space in accordance with the detected CO₂ gas concentration.

4. The CO₂ incubator according to claim 3, wherein the control means (12) displays the CO₂ gas concentration detected in each incubation space.

5. The CO₂ incubator according to any preceding claim, wherein the control means (11) is configured to execute an operation of proportion and integration.

6. The CO₂ incubator according to any preceding claim, wherein the control means (11) is configured to execute an operation of proportion, integration and differentiation.

7. A CO₂ gas controller (11) for controlling supply of CO₂ gas to a given space, the controller comprising:
means for receiving a value of CO₂ gas concentration in said given space; and
means for outputting a signal to means (9) for supplying CO₂ gas to said given space;
said controller being configured to execute an operation of proportion, proportion and integration, or proportion, integration and differentiation on the basis of a difference between said value of CO₂ gas concentration and a set value of CO₂ gas concentration so thereby to determine a CO₂ gas supply time and stop time and to output control signals in accordance with said supply time and stop time, wherein said proportion operation is for calculating a control amount in proportion to the deviation for reducing the deviation, said integral operation is for calculating a control amount for reducing an integrated value of the deviation and said differential operation is for calculating a control amount for reducing a differentiated value of the deviation.

8. A method of controlling supply of CO₂ gas to a given space, the method comprising:
receiving a value of CO₂ gas concentration in said given space;
executing an operation of proportion, proportion and integration, or proportion, integration and differentiation on the basis of a difference between the measured value of CO₂ gas concentration and a set value of CO₂ gas concentration so thereby to determine a CO₂ gas supply time and stop time;
outputting control signals to means for supplying CO₂ gas to said given space in accordance with said supply time and stop time,
wherein said proportion operation is for calculating a control amount in proportion to the deviation for reducing the deviation, said integral operation is for calculating a control amount for reducing an integrated value of the deviation and said differential operation is for calculating a control amount for reducing a differentiated value of the deviation.

## Patentansprüche

1. CO₂-Inkubator (1) zum Inkubieren eines Kulturmediums, das in einem Inkubationsraum (S) untergebracht ist, der in einem Speicherraum definiert ist, wobei der CO₂-Inkubator (1) umfaßt:
eine CO₂-Gaskonzentration-Detektionseinrichtung (6) zum Detektieren einer CO₂-Konzentration im Inkubationsraum (S);
eine CO₂-Gaszuführeinrichtung (8, 9, 10) zum Zuführen von CO₂-Gas in den Inkubationsraum (S); und
eine Steuereinrichtung (11) zum Steuern der CO₂-Gaszuführeinrichtung (9),
**gekennzeichnet durch**:
eine CO₂-Gaskonzentration-Einstelleinrichtung (12) zum Einstellen einer CO₂-Gaskonzentration;
wobei die Steuereinrichtung (11) dazu konfiguriert ist, auf Grundlage einer Abweichung zwischen der detektierten CO₂-Gaskonzentration und dem eingestellten CO₂-Gaskonzentrationswert eine Proportion-, eine Proportion- und Integration-, oder eine Proportion- und Integration- und Differenzierungs-Operation durchzuführen, um eine CO₂-Gaszuführzeit zum Inkubationsraum pro Zeiteinheit sowie eine Stoppzeit zu berechnen, wobei die Proportion-Operation in Proportion zur Abweichung eine Steuermenge berechnet, um die Abweichung zu reduzieren, die Integraloperation eine Steuermenge zum Reduzieren eines integrierten Wertes der Abweichung berechnet, und die Differenzierungsoperation eine Steuermenge zum Reduzieren eines differenzierten Wertes der Abweichung berechnet; und
wobei die Steuereinrichtung (11) so konfiguriert ist, dass sie die Zufuhr von CO₂-Gas von der CO₂-Gaszuführeinrichtung (8, 9, 10) zum Inkubationsraum (S) in Übereinstimmung mit der berechneten Zuführzeit und der Stoppzeit steuert.

2. CO₂-Inkubator (1) nach Anspruch 1, wobei die CO₂-Gaskonzentration-Detektionseinrichtung (6) aus einem CO₂-Sensor besteht, der Infrarotstrahlen verwendet.

3. CO₂-Inkubator nach Anspruch 1 oder 2, wobei eine Mehrzahl von Inkubationsräumen (S1, S2) angeordnet sind und die Steuereinrichtung (25) das Gas in einem beliebigen Inkubationsraum selektiert, die CO₂-Gaskonzentration des selektierten Gases mittels der CO₂-Gaskonzentration-Detektionseinrichtung (6) detektiert und die Zufuhr des CO₂-Gases an jeden Inkubationsraum in Übereinstimmung mit der detektierten CO₂-Gaskonzentration steuert.

4. CO₂-Inkubator nach Anspruch 3, wobei die Steuereinrichtung (12) die in jedem Inkubationsraum detektierte CO₂-Gaskonzentration anzeigt.

5. CO₂-Inkubator nach einem der vorhergehenden Ansprüche, wobei die Steuereinrichtung (11) dazu konfiguriert ist, eine Proportion- und Integration-Operation auszuführen.

6. CO₂-Inkubator nach einem der vorhergehenden Ansprüche, wobei die Steuereinrichtung (11) dazu konfiguriert ist, eine Proportion-, Integration- und Differenzierungs-Operation auszuführen.

7. CO₂-Gas-Steuereinrichtung (11) zum Steuern der Zufuhr von CO₂-Gas an einen vorbestimmten Raum, wobei die Steuereinrichtung umfaßt:
eine Einrichtung zum Empfangen eines CO₂-Gaskonzentrationswertes in dem vorbestimmten Raum; und
eine Einrichtung zum Ausgeben eines Signals an eine Einrichtung (9) zum Zuführen von CO₂-Gas an den vorbestimmten Raum;
wobei die Steuereinrichtung dazu konfiguriert ist, auf Grundlage einer Differenz zwischen dem CO₂-Gaskonzentrationswert und einem eingestellten CO₂-Gaskonzentrationswert eine Proportion-, eine Proportion- und Integration-, oder eine Proportion- und Integration- und Differenzierungs-Operation durchzuführen, um so eine CO₂-Gaszuführzeit sowie eine Stoppzeit zu ermitteln und in Übereinstimmung mit der Zuführzeit und der Stoppzeit Steuersignale auszugeben, wobei die Proportion-Operation zur Berechnung einer Steuermenge in Proportion zur Abweichung dient, um die Abweichung zu reduzieren, die Integral-Operation zum Berechnen einer Steuermenge dient, um einen integrierten Wert der Abweichung zu reduzieren, und die Differenzierungsoperation zum Berechnen einer Steuermenge dient, um einen differenzierten Wert der Abweichung zu reduzieren.

8. Verfahren zum Steuern der Zufuhr von CO₂-Gas an einen vorbestimmten Raum, wobei das Verfahren umfaßt:
Empfangen eines CO₂-Gaskonzentrationswertes im vorbestimmten Raum;
Ausführen einer Proportion-, einer Proportion- und Integration-, oder einer Proportion-, Integration- und Differenzierungs-Operation auf Grundlage einer Differenz zwischen dem gemessenen CO₂-Gaskonzentrationswert und einem eingestellten CO₂-Gaskonzentrationswert, um so eine CO₂-Gas-Zuführzeit und eine Stoppzeit zu ermitteln;
Ausgeben von Steuersignalen an eine Einrichtung zum Zuführen von CO₂-Gas an den vorbestimmten Raum in Übereinstimmung mit der Zuführzeit und der Stoppzeit,
wobei die Proportion-Operation zum Berechnen einer Steuermenge in Proportion zur Abweichung dient, um die Abweichung zu reduzieren, die Integral-Operation zum Berechnen einer Steuermenge dient, um einen integrierten Wert der Abweichung zu reduzieren, und die Differenzierungs-Operation zum Berechnen einer Steuermenge dient, um einen differenzierten Wert der Abweichung zu reduzieren.

## Revendications

1. Incubateur (1) à CO₂ destiné à incuber un milieu de culture logé dans un espace (S) d'incubation défini dans une chambre de stockage, l'incubateur (1) à CO₂ comprenant :
un moyen (6) de détection de concentration de CO₂ gazeux destiné à détecter une concentration de CO₂ dans l'espace (S) d'incubation ;
un moyen (8, 9, 10) de délivrance de CO₂ gazeux destiné à délivrer du CO₂ gazeux dans l'espace (S) d'incubation ; et
un moyen (11) de commande destiné à commander le moyen (9) de délivrance de CO₂ gazeux,
**caractérisé :**
**par** un moyen (12) de paramétrage de concentration de CO₂ gazeux destiné à paramétrer une concentration de CO₂ gazeux ;
en ce que le moyen (11) de commande est constitué pour exécuter une opération de proportion, de proportion et d'intégration, ou de proportion et d'intégration et de différentiation sur la base d'un écart entre la concentration détectée de CO₂ gazeux et la valeur paramétrée de concentration de CO₂ gazeux pour calculer un temps de délivrance, à l'espace d'incubation, de CO₂ gazeux par unité de temps et un temps d'arrêt, dans lequel ladite opération de proportion calcule une quantité de commande en proportion de l'écart pour réduire l'écart, ladite opération d'intégration calcule une quantité de commande pour réduire une valeur intégrée de l'écart et ladite opération de différentiation calcule une quantité de commande pour réduire une valeur différentiée de l'écart ; et
en ce que le moyen (11) de commande est constitué pour commander la délivrance de CO₂ gazeux à l'espace (S) d'incubation par le moyen (8, 9, 10) de délivrance de CO₂ gazeux en fonction du temps de délivrance et du temps d'arrêt, calculés.

2. Incubateur (1) à CO₂ selon la revendication 1, dans lequel le moyen (6) de détection de concentration de CO₂ gazeux est constitué d'un capteur de CO₂ utilisant des rayons infrarouges.

3. Incubateur à CO₂ selon la revendication 1 ou 2, dans lequel il est prévu une pluralité d'espaces (S1, S2) d'incubation et dans lequel le moyen (25) de commande choisit le gaz dans l'un quelconque des espaces d'incubation, détecte, à l'aide du moyen (6) de détection de concentration de CO₂ gazeux, la concentration de CO₂ gazeux du gaz choisi, et commande la délivrance du CO₂ gazeux à chaque espace d'incubation en fonction de la concentration détectée de CO₂ gazeux.

4. Incubateur à CO₂ selon la revendication 3, dans lequel le moyen (12) de commande affiche la concentration de CO₂ gazeux détectée dans chaque espace d'incubation.

5. Incubateur à CO₂ selon l'une quelconque des revendications précédentes, dans lequel le moyen (11) de commande est constitué pour exécuter une opération de proportion et d'intégration.

6. Incubateur à CO₂ selon l'une quelconque des revendications précédentes, dans lequel le moyen (11) de commande est constitué pour exécuter une opération de proportion, d'intégration et de différentiation.

7. Régulateur (11) de CO₂ gazeux destiné à commander la délivrance de CO₂ gazeux à un espace donné, le régulateur comprenant :
un moyen destiné à recevoir une valeur de concentration de CO₂ gazeux dans ledit espace donné ; et
un moyen destiné à sortir un signal vers un moyen (9) destiné à délivrer du CO₂ gazeux audit espace donné ;
ledit régulateur étant constitué pour exécuter une opération de proportion, de proportion et d'intégration, ou de proportion, d'intégration et de différentiation sur la base d'une différence entre ladite valeur concentration de CO₂ gazeux et une valeur paramétrée de concentration de CO₂ gazeux pour déterminer ainsi un temps de délivrance de CO₂ gazeux et un temps d'arrêt, et pour sortir des signaux de commande en fonction desdits temps de délivrance et temps d'arrêt, dans lequel ladite opération de proportion sert à calculer une quantité de commande en proportion de l'écart pour réduire l'écart, ladite opération d'intégration sert à calculer une quantité de commande pour réduire une valeur intégrée de l'écart et ladite opération de différentiation sert à calculer une quantité de commande pour réduire une valeur différentiée de l'écart.

8. Procédé de commande de délivrance de CO₂ gazeux à un espace donné, le procédé comprenant :
la réception d'une valeur de concentration de CO₂ gazeux dans ledit espace donné ;
l'exécution d'une opération de proportion, de proportion et d'intégration, ou de proportion, d'intégration et de différentiation sur la base d'une différence entre la valeur mesurée de concentration de CO₂ gazeux et une valeur paramétrée de concentration de CO₂ gazeux pour déterminer ainsi un temps de délivrance de CO₂ gazeux et un temps d'arrêt ;
la sortie de signaux de commande vers un moyen destiné à délivrer du CO₂ gazeux audit espace donné en fonction desdits temps de délivrance et temps d'arrêt,
dans lequel ladite opération de proportion sert à calculer une quantité de commande en proportion de l'écart pour réduire l'écart, ladite opération d'intégration sert à calculer une quantité de commande pour réduire une valeur intégrée de l'écart et ladite opération de différentiation sert à calculer une quantité de commande pour réduire une valeur différentiée de l'écart.
